# EUROPEAN PATENT APPLICATION

(11) **EP 2 138 168 A1**
(43) Date of publication of application: **30.12.2009**
(21) Application number: 08720926.8
(22) Date of filing: 27.02.2008
(51) Int. Cl.: A61K 9/70, A61J 3/07, A61K 9/00

(54) **AGENT FOR ORAL ADMINISTRATION AND METHOD FOR PRODUCING THE SAME**

(30) Priority: 30.03.2007 JP 2007091803
(71) Applicant: Lintec Corporation, Itabashi-ku Tokyo 173-0001 (JP)
(72) Inventor: SUGIURA, Yusaku, Tokyo 173-0001 (JP); YANAGIMOTO, Kaisuke, Tokyo 173-0001 (JP); SUZUKI, Eiji, Tokyo 173-0001 (JP)
(74) Representative: Epping - Hermann - Fischer
(86) International application number: PCT/JP2008/053378
(87) International publication number: WO 2008/126488

(57) **Abstract**

Film-like orally administered medications are provided which are less likely to be attached to the oral mucous membrane upon intake and can be easily detached therefrom even if attached thereto. A film-like orally administered medication (1) includes at least a medical-agent-containing layer (11). Convex portions are formed on at least one surface of the orally administered medication (1). The convex portions have at least one type of shape selected from among tapers, columns, hemispheres, and frustums. The medical-agent-containing layer includes a medical agent and a base substance.

## Description

### [Field of the Invention]

The present invention relates to a film-like orally administered medication and a manufacturing method therefor.

### [Background Art]

Orally administered medications commonly take the form of solid medications such as tablets, capsules, or the like. Because people have difficulty in directly swallowing such solid medications, they often have to take the medications with plenty of water. Even if they do so, some of them may still find it difficult to swallow, which may result in poor medication compliance. When solid medications entail such swallowing difficulties, it is also likely that people clog their tracheae with the solid medications by mistake or that the solid medications are attached to part of their esophagi, resulting in formation of esophageal tumors on that part.

Because especially the elderly and infants are more likely to experience difficulty in swallowing solid medications, the use of jelly-like, semisolid medications is desired in order to avoid the swallowing difficulties associated with the solid medications and enhance the ease and safety during intake. Such jelly-like, semisolid medications, however, contain a larger amount of water; accordingly, they are subject to decrease in the stability of their medical agents (especially those that are easily hydrolyzed).

For this reason, it has been proposed to process medications into films (or sheets) to enhance the stability of their medical agents (for example, refer to Patent Document 1 below). When medications are processed into films, the water contents of the medications can be suppressed. This leads to improvement in the stability of the medical agents (especially those that are prone to hydrolysis) in the medications and also allows for easy handling of the medications and reduction in packaging costs.
Patent Document 1: international publication WO2002/087622 (refer to claims 1 and 2 and FIG. 1)

### [Disclosure of the Invention]

### [Problems to be Solved by the Invention]

When such a film-shaped medication is taken, it may occasionally be attached to the oral mucous membrane, especially that of the upper jaw during intake. Once attached, the medication cannot be detached with ease.

In view of the above problem, the invention thus aims to provide film-shaped orally administered medications which are less likely to be attached to the oral mucous membrane upon intake and can be easily detached therefrom even if attached thereto and to provide a manufacturing method therefor.

### [Means for Solving the Problems]

According to a first aspect of the present invention, a film-like orally administered medication comprises at least a medical-agent-containing layer, wherein a convex portion is formed on at least one surface of the orally administered medication. The formation of the convex portion allows the top surface of the convex portion to come into direct contact with the oral mucous membrane, making the contact area of the orally administered medication with the oral mucous membrane smaller. Accordingly, even if attached to the oral mucous membrane during intake, the orally administered medication can be detached with ease.

Preferably, the orally administered medication comprises a gel forming layer as an outermost layer of the orally administered medication, and a convex portion is formed on the surface of the gel forming layer. If the orally administered medication is slow in gelation, it is more likely to be attached to the oral mucous membrane. To avoid this, the convex portion is provided to increase the entire surface area of the orally administered medication, then facilitate the gelation process, and make the orally administered medication less likely to be attached to the oral mucous membrane.

Preferably, the convex portion has at least one type of shape selected from among tapers, columns, hemispheres, and frustums. Preferably, the height of the convex portion is in the range from 10 to 5,000 µm. Preferably, the convex portion is provided in the form of a multiplicity of convex portions on at least one surface of the orally administered medication.

According to the orally administered medication of the present invention, preferably the medical-agent-containing layer includes a medical agent and a base substance. Preferably, the thickness of the medical-agent-containing layer is in the range from 0.1 to 1,000 µm. Preferably, the gel forming includes a water-swellable gel forming agent and a film-forking agent. Preferably, the thickness of the gel forming layer including the thickness of the convex portion is in the range from 20 to 6,000 µm.

According to a second aspect of the present invention, a method for manufacturing an orally administered medication, comprises the steps of: applying a coating liquid for a gel forming layer onto a base film having a concave portion formed thereon and drying; applying a coating liquid for a medical-agent-containing layer onto the gel forming layer and drying, thus forming a laminated body having the laminated medical-agent-containing layer/ gel forming layer/base film in the described order; preparing two of the laminated body and joining and pressure-bonding the two laminated bodies together with the medical-agent-containing layers thereof facing each other; and removing the base films and cutting the rest into a predetermined shape to form the orally administered medication.

According to a third aspect of the present invention, a method for manufacturing an orally administered medication, comprises the steps of: applying a coating liquid for a first gel forming layer onto a first base film having a concave portion formed thereon and drying to form the first gel forming layer; printing a coating liquid for a medical-agent-containing layer onto the first gel forming layer and drying to form the medical-agent-containing layer that is smaller than the first gel forming layer; applying coating a liquid for a second gel forming layer onto a second base film having a concave portion formed thereon and drying to form the second gel forming layer; and bonding peripheral edges of the first and second gel forming layers together to form the orally administered medication.

### [Effect of the Invention]

The orally administered medication according to the first aspect of the invention is, even if film-shaped, less likely to be attached to the oral mucous membrane and can be easily detached therefrom even if attached thereto, which leads to better medication compliance.

According to the second and third aspects of the invention, it is possible to provide orally administered medications which are less likely to be attached to the oral mucous membrane, can be easily detached therefrom even if attached thereto, and lead to better medication compliance.

### [Brief Description of the Drawings]

FIG. 1 is a cross-sectional schematic of an orally administered medication according to the invention;
FIGS. 2A and 2B are schematics illustrating the structure of convex portions according to a first embodiment which are formed on an surface of the orally administered medication of the invention;
FIGS. 3A and 3B are schematics illustrating the structure of convex portions according to a second embodiment which are formed on an surface of the orally administered medication of the invention;
FIGS. 4A and 4B are schematics illustrating the structure of convex portions according to a third embodiment which are formed on an surface of the orally administered medication of the invention;
FIG. 5 is a schematic illustrating the structures of convex portions according to a fourth embodiment which are formed on an surface of the orally administered medication of the invention; and
FIG. 6 is a cross-sectional schematic of a second orally-administered medication according to the invention.

### [Description of Reference Numerals]

- 1: orally administered medication
- 2: orally administered medication
- 11: medical-agent-containing layer
- 12: gel forming layer
- 13: gel forming layer
- 21: first gel forming layer
- 22: second gel forming layer
- 23:: medical-agent-containing layer
- 24:: surface of gel forming layer

### [Best Mode for Carrying out the Invention]

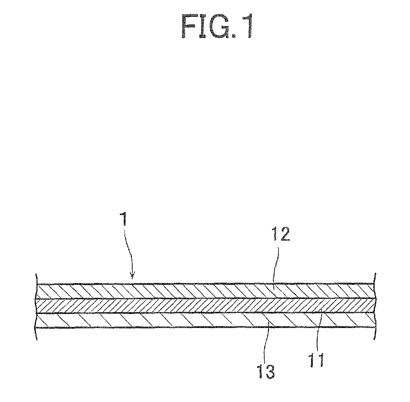

An orally administered medication according to the invention is now described with reference to FIG. 1.

The orally administered medication according to the invention, designated 1, takes the form of a film that includes at least a single medical-agent-containing layer 11. The orally administered medication 1 of the invention may include a single medical-agent-containing layer 11 or may include a multiplicity of medical-agent-containing layers. In the latter case, different medical-agent-containing layers 11 may contain different medical agents. When the orally administered medication 1 of the invention includes a multiplicity of medical-agent-containing layers 11, those may be stacked directly on one another or stacked via an adhesive layer. Alternatively, those can be connected to one another in a direction perpendicular to the stacking direction to form a single medical-agent-containing layer; in this case, too, the layers can be connected via the adhesive layer.

At least either the upper face or lower face of a single medical-agent-containing layer 11, which may consist of a multiplicity of medical-agent-containing layers 11, is preferably covered with a gel forming layer. It is particularly preferred to provide water-swellable gel forming layers 12 and 13 on the upper face and lower face, respectively, of the single medical-agent-containing layer 11 as the outermost layers of the orally administered medication 1. Once inside the oral cavity of the patient, the water-swellable gel forming layers provided as the outermost layers of the orally administered medication swell in response to water such as saliva and the like and turn into a gel, thus turning the orally administered medication into a dosage form that has the appropriate size, shape, elasticity, and viscosity for swallowing. Thus, the patient can take the orally administered medication with ease. Even if the patient is an elderly person or an infant, they can take the orally administered medication safely due to reduced risk of the orally administered medication getting stuck in patient's trachea during intake. If the patient is unable to secrete saliva enough to turn the water-swellable gel forming layers into a complete gel, the same effect can be achieved by having the patient take the orally administered medication with a small amount of water or by soaking the medication in water in advance before dosage. The amounts of water required in those cases are much smaller than that required for taking solid medications such tablets, capsules, or the like. Further, after the water-swellable gel forming layers provided as the outermost layers of the orally administered medication swell in response to water such as saliva and the like and turn into a gel in the oral cavity of the patient, the gel serves to mask the tastes (for example, bitterness and astringency) and smells of the medical agents contained in the medical-agent-containing layer.

Convex portions are formed at least either on the surface of the gel forming layer 12 or on the surface of the gel forming layer 13. If the orally administered medication is slow in gelation, it is more likely to be attached to the oral mucous membrane. To avoid this, the convex portions are provided to increase the surface area of the orally administered medication, facilitate the gelation process, and make the orally administered medication less likely to be attached to the oral mucous membrane. Also, because the formation of the convex portions decreases the direct contact area of the surface of the orally administered medication with the oral mucous membrane, the orally administered medication can be detached easily from the oral mucous membrane even if attached thereto during intake. In this case, it is preferred that the convex portions be provided on both surfaces of the gel forming layers 12 and 13, which serves as the outermost layers of the orally administered medication 1. This is so because there is less chance of the orally administered medication being attached to the oral mucous membrane of the upper jaw even if it is taken with either side up and because it can be easily detached therefrom even if attached thereto.

The convex portions can have any shape as long as it reduces the contact areas of the convex portions with the oral mucous membrane; it can be a shape that allows plane contact or point contact. For example, possible shapes of the convex portions include: tapers; columns; hemispheres frustums; and the like. The tapers include: cones whose bases are circular or ellipsoidal; pyramids; substantial cones; and substantial pyramids. The columns include substantial columns, for example, columns whose top faces are curved. The hemispheres include those that contain curved surfaces, those that are cut partially, and the like. The frustums include: truncated pyramids, truncated cones whose bases are circular or ellipsoidal; substantial frustums; and also include not only those that are formed by truncating a taper by a plane parallel to its base but those that are formed by truncating a taper by a plane that is not parallel to its base.

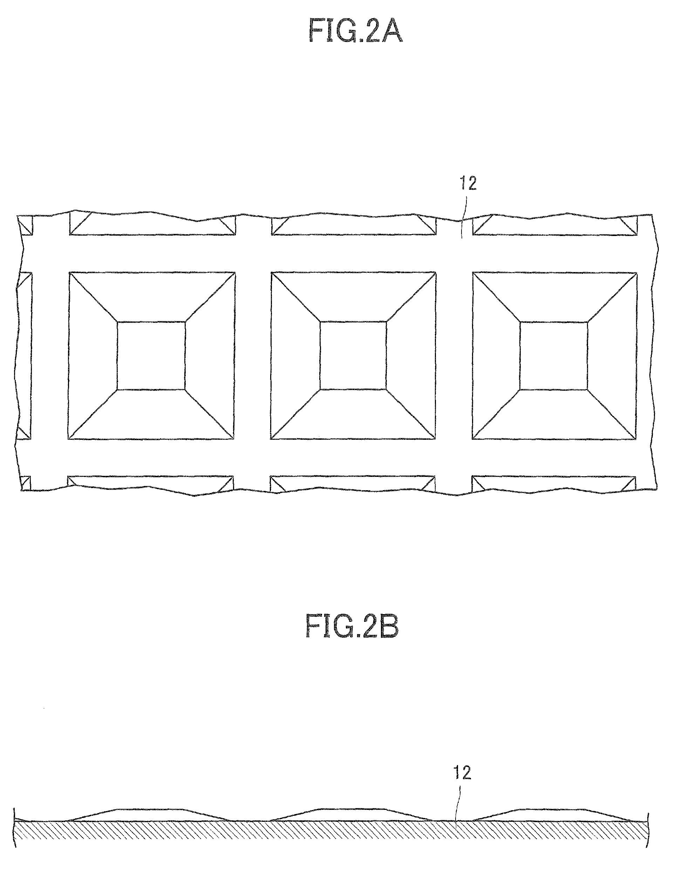

When only one convex portion is provided, the entire surfaces of the orally administered medication 1 excluding that convex portion are likely to come into contact with the oral mucous membrane. In that case, flow paths for saliva and the like would not be formed on the surfaces of the orally administered medication 1. This makes the orally administered medication 1 less gellable due to lack of water supply. As a result, the orally administered medication 1 may not be easily detached from the oral mucous membrane of the upper jaw once attached. Therefore, it is preferred to form a multiplicity of convex portions on the entire surfaces of the orally administered medication 1. The multiplicity of convex portions can be provided, for example, linearly or in a polka-dot pattern. By thus providing the multiplicity of convex portions, the spaces between the convex portions do not come into direct contact with the oral mucous membrane, functioning as saliva flow paths. These saliva flow paths secure an adequate water supply for gelation and facilitate the gelation. As a result, even if the orally administered medication is attached to the oral mucous membrane, it can be detached more easily. Especially preferred is to form a great number of convex portions in a given microscale pattern in which each of the convex portions is, for example, on the order of micrometers. FIGS. 2A to 5 illustrate preferred examples of convex portions formed. The formation of such convex portions as shown in FIGS. 2A to 5 allows the top surface of each convex portion to come into contact with the oral mucous membrane. In this case, the overall contact area of the orally administered medication with the oral mucous membrane is smaller than that of a flat, sheet-like medication, and flow paths for saliva and the like can be secured. Thus, even if the orally administered medication is attached to the oral mucous membrane, it can be detached with ease. In addition, because the surface area of the orally administered medication increases, the gelation time can be shortened, which makes the medication less likely to be attached to the oral mucous membrane.

FIGS. 2A and 2B are to explain the shapes of convex portions according to a first embodiment. FIG. 2A is a partially enlarged view of the gel forming layer 12 on which are formed the multiplicity of convex portions according to the first embodiment; and FIG. 2B is its cross sectional view. The convex portions according to the first embodiment are truncated pyramids whose bases are square in shape, and the top surfaces of the truncated pyramids come into contact with the oral mucous membrane. The size of each of the convex portions of the first embodiment shown in FIGS. 2A and 2B is such that, for example, one side of its square base is 450 µm long, one side of its top square surface is 184 µm long, and the height between the base and the top surface is 30 µm.

These convex portions according to the first embodiment can be formed in a regular pattern over the entire surface of the gel forming layer 12 or in a random pattern. The exemplary formation pattern of the convex portions shown in FIGS. 2A and 2B is a grid in which the convex portions are arranged at 100-µm intervals.

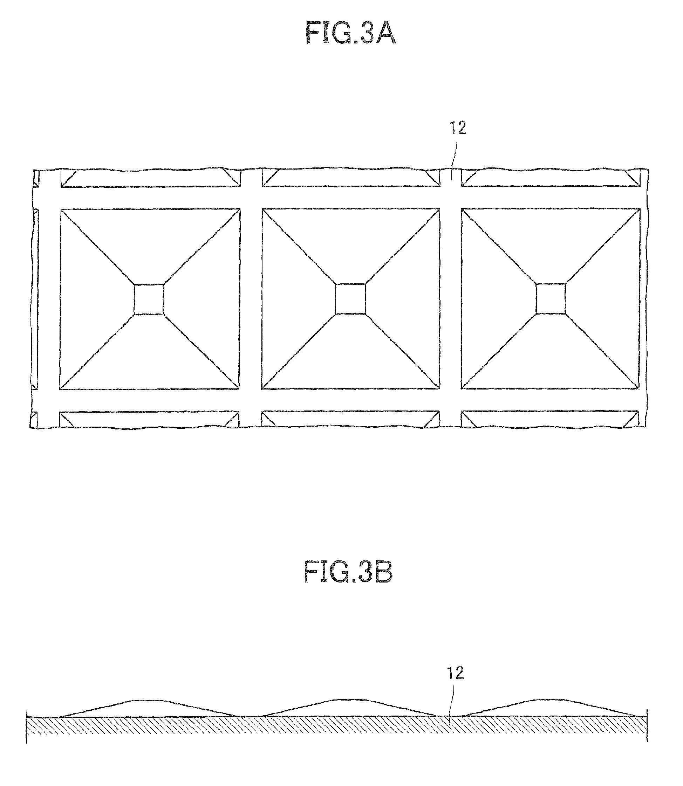

FIGS. 3A and 3B are to explain the shapes of convex portions according to a second embodiment. FIG. 3A is a partially enlarged view of the gel forming layer 12 on which are formed two or more of the convex portions according to the second embodiment, and FIG. 3B is its cross sectional view. The convex portions according to the second embodiment are also truncated pyramids whose bases are square in shape, and the top surfaces of the truncated pyramids come into contact with the oral mucous membrane. The size of each of the convex portions of the second embodiment shown in FIGS. 3A and 3B is such that, for example, one side of its square base is 600 µm long, one side of its top square surface is 100 µm long, and the height between the base and the top surface is 66 µm.

These convex portions according to the second embodiment can also be formed in a regular pattern over the entire surface of the gel forming layer or in a random pattern. The exemplary formation pattern of the convex portions shown in FIGS. 3A and 3B is a grid in which the convex portions are arranged at 75-µm intervals.

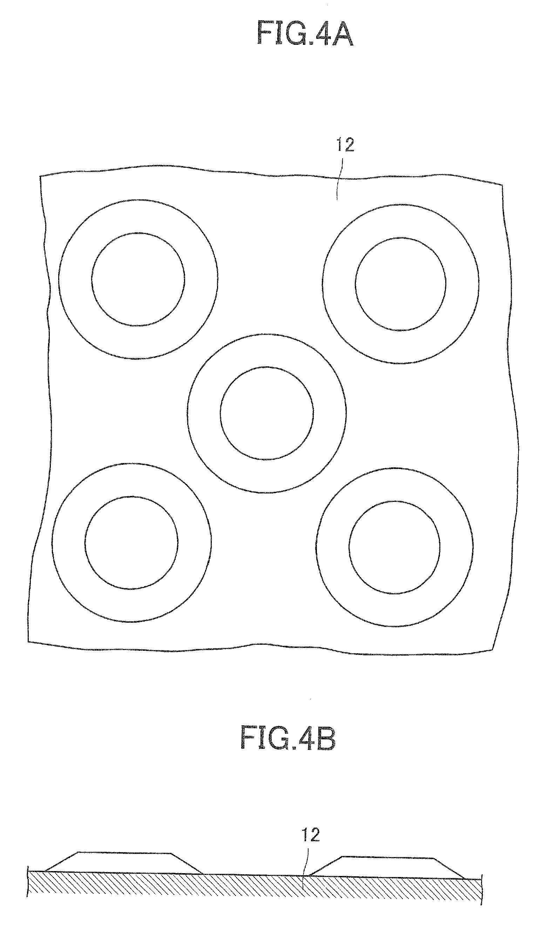

FIGS. 4A and 4B are to explain the shapes of convex portions according to a third embodiment. FIG. 4A is a partially enlarged view of the gel forming layer 12 on which are formed two or more of the convex portions according to the third embodiment, and FIG. 4B is its cross-sectional view. The convex portions according to the third embodiment are truncated cones, and the top surfaces of the truncated cones come into contact with the oral mucous membrane. The size of each of the convex portions of the third embodiment shown in FIGS. 4A and 4B is such that, for example, the diameter of its base is 1,750 µm, the diameter of its top surface is 1,000 µm, and the height is 183 µm. These convex portions can also be formed in a regular pattern over the entire surface of the gel forming layer or in a random pattern. The exemplary formation pattern of the convex portions shown in FIGS. 4A and 4B is a checkered pattern.

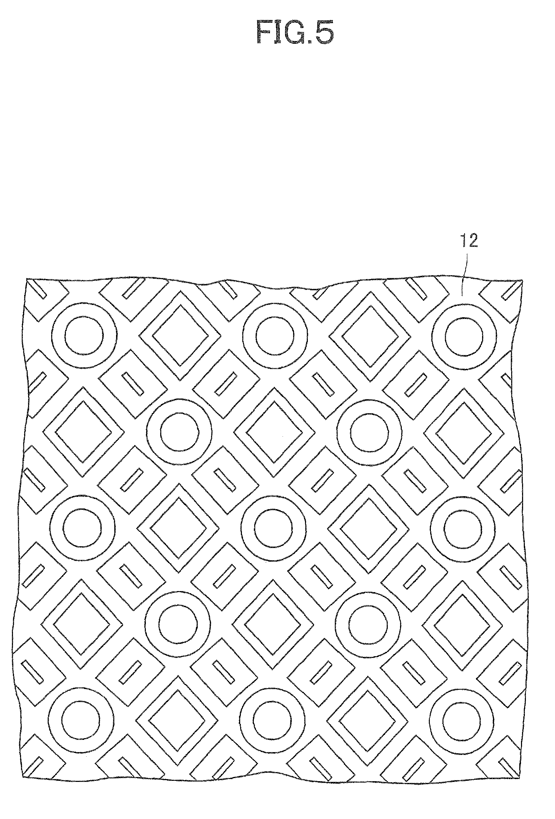

Further, as shown by the convex portions of FIG. 5 according to a fourth embodiment, convex portions of two or more types (three types in FIG. 5) can be formed on the surface of the gel forming layer 12. The convex portions of FIG. 5 include the ones of the third embodiment shown in FIGS. 4A and 4B, truncated-pyramid-shaped convex portions whose bases are rectangular, and truncated-pyramid-shaped convex portions whose bases are rhombus-shaped. The size of each of the truncated-pyrazuid-shaped convex portions with rectangular bases is such that, for example, its base is 1,500 µm×1,150 µm in area, its top surface is 750 µmx150 µm in area, and the height is 183 µm. The size of each of the truncated-pyramid- shaped convex portions with rhombus-shaped bases is such that, for example, its base is 1,680 µm×1,680 µm in area, its top surface is 1,150 µm×1,150 µm in area, and the height is 23 µm. Those convex portions of the two types (truncated-pyramid shape) can differ in height as above or be of the same height. When two or more types of convex portions are formed as shown in FIG. 5, they can be formed in a regular pattern or in a random pattern.

Preferably, the heights of the convex portions are in the range from 10 to 5,000 µm and more preferably in the range from 20 to 1,000 µm. The reasons are that difficulty is involved in forming convex portions of more than 5,000 µm in height and that formation of convex portions of less than 10 µm in height makes the whole area of the surface on which to form the convex portions more likely to be attached to the oral mucous membrane. Also, it is preferred to form convex portions such that there are 1 to 10,000 convex portions per 100 mm². Further, when the top surfaces of convex portions are planar, the total area of the top surfaces of the convex portions of the film-like orally administered medication is preferably 0.01 to 30 %, more preferably 0.1 to 20 %, of the surface area on which the convex portions are formed. When the total area above is in those ranges, favorable orally-administered medications can be obtained which ensures better medication compliance.

The thickness of the medical-agent-containing layer 11 can be adjusted as desired according to the amount of the medical agents and within the thickness range in which the medication can be administered orally. Preferably, the thickness is in the range from 0.1 to 1,000 µm and more preferably in the range from 10 to 200 µm. When the thickness of the medical-agent-containing layer 11 is less than 0.1 µm, the medical agent content may vary in whole the layer 11. When, on the other hand, the thickness of the medical-agent-containing layer 11 is more than 1,000 µm, the patient may have difficulty in taking such a thick medication.

The thickness of each of the gel forming layers 12 and 13 including convex portions formed thereon is preferably in the range from 20 to 6,000 µm and more preferably in the range from 30 to 2,000 µm. When the thickness is less than 20 µm, the orally administered medication 1 is less likely to change into a dosage form that has the appropriate size, shape, elasticity, and viscosity for swallowing. When, on the other hand, the thickness is more than 6,000 µm, the patient may have difficulty in taking such a thick medication.

Although the medical-agent-containing layer 11 may include only medical agents, it is preferred that the medical-agent-containing layer 11 also include base substances that serve to maintain the medical agents in a desired state. The medical-agent-containing layer 11 can include a variety of medical agents ranging from those administered in small quantities (for example, a dose of 1 mg or less) to those administered in large quantities (for example, a dose of 300 mg or more). Further, the medical-agent-containing layer 11 can include plasticizers to attain its appropriate flexibility.

The gel forming layers 12 and 13 include at least water-swellable substances, that is, water-swellable gel forming agents that swell in response to water to form a gel. However, when it is difficult to manufacture film-like medication by using only those water-swellable gel forming agents in the gel forming layers 12 and 13, it is preferred to add film-forming agents to the gel forming layers 12 and 13. Further, the gel forming layers can include plasticizers to attain its appropriate flexibility.

The medical-agent-containing layer 11 and the gel forming layers 12 and 13 can also include: masking agents; colorants; preservatives; and the like. When orally administered medications are to be manufactured which include only medical-agent-containing layers, it is preferred to use, as the base substances of the medical-agent-containing layers 11, substances that function also as water-swellable gel forming agents; the selection of which substances to add can be performed according to intended purposes.

Hereinafter, substances that can be included in the aforementioned medical-agent-containing layer 11 and gel forming layers 12 and 13 are discussed in detail.

### (Medical agents)

Medical agents are not limited to particular substances as long as the medical agents are those that should be administered to the patient and can be administered orally. Examples of medical agents that can be administered orally include: medical agents that act on central nervous system; medical agents that act on respiratory system; medical agents that act on circulatory system; hematologic and hematogenic agents; medical agents that act on digestive system; and medical agents that work for metabolic diseases. The medical agents that act on the central nervous system include: hypnotic agents such as amobarbital, estazolam, triazolam, nitrazepam, pentobarbital, and the like; psychotropic agents such as amitriptyline hydrochloride, imipramine hydrochloride, oxazolam, chlordiazepoxide, chlorpromazine, diazepam, sulpiride, haloperidol, and the like; antiparkinsonian agents such as trihexyphenidyl, levodopa, and the like; sedative agents and anti-inflammatory agents such as aspirin, isopropylantipyrine, indomethacin, diclofenac sodium, mefenamic acid, streptokinase, streptodornase, serrapeptase, pronase, dipotassium glycyrrhizate, disodium glycyrrhizinate, and the like; and metabolic activators for the central nervous system such as ATP, vinpocetine, and the like. The medical agents that act on the respiratory system include: expectorants such as carbocisteine, bromhexine hydrochloride, and the like; and antiasthmatic agents such as azelastine hydrochloride, oxatomide, theophylline, terbutaline sulfate, tranilast, procaterol hydrochloride, ketotifen fumarate, and the like. The medical agents that act on the circulatory system includes cardiotonic agents such as aminophylline, digitoxin, digoxin, and the like; antiarrhythmic agents such as ajmaline, disopyramide, procainamide hydrochloride, mexiletine hydrochloride, and the like; antianginal agents such as amyl nitrite, alprenolol hydrochloride, isosorbide dinitrate, nicorandil, oxyfedrine, dipyridamole, dilazep hydrochloride, diltiazem hydrochloride, nitroglycerin, nifedipine, verapamil hydrochloride, and the like; peripheral vasodilators such as kallidinogenase and the like; antihypertensive agents such as atenolol, captopril, clonidine hydrochloride, metoprolol tartrate, spironolactone, triamterene, trichlormethiazide, nicardipine, hydralazine hydrochloride, hydrochlorothiazide, prazosin hydrochloride, furosemide, propranolol hydrochloride, enalapril maleate, methyldopa, labetalol hydrochloride, reserpine, and the like; and antiarteriosclerotic agents such as clofibrate, dextran sulfate, nicomol, niceritrol, and the like. The hematologic and hematogenic agents include: hemostatic agents such as carbazochrome sodium sulfonate, tranexamic acid, and the like; antithrombogenic agents such as ticlopidine hydrochloride, warfarin potassium, and the like; and antianemia agents such as ferric sulfate and the like. The medical agents that act on the digestive system include: antiulcer agents such as azulene, aldioxa, cimetidine, ranitidine hydrochloride, famotidine, teprenone, rebamipide, and the like; antiemetic agents such as domperidone, metoclopramide, and the like; cathartics such as sennoside and the like; digestive enzyme agents; and agents for hepatic diseases such as glycyrrhizin, liver-extract-containing agents, and the like. The medical agents that work for metabolic diseases include- antidiabetic agents such as glibenclamide, chlorpropamide, tolbutamide, and the like; and antipodagric agents such as allopurinol, colchicines, and the like. Further, examples of medical agents that can be administered orally include: medical agents used in the field of ophthalmology such as acetazolamide and the like; medical agents used in the field of otolaryngology including antivertigo agents such as difenidol hydrochloride, betahistine mesylate, and the like; chemomedical agents and antibiotics such as isoniazid, ethambutol, hydrochloride, ofloxacin, erythromycin stearate, cefaclor, norfloxacin, fosfomycin calcium, minocycline hydrochloride, rifampicin, rokitamycin, and the like; anticancer agents such as cyclophosphamide, tegafur, and the like; immunosuppressive agents such as azathioprine and the like; hormones and agents for endocrine secretion such as progestational hormones, salivary gland hormones, thiamazole, prednisolone, betamethasone, liothyronine, levothyroxine, and the like; and autacoids. The autacoids include: antihistamine agents such as clemastine fumarate, d-chlorpheniramine maleate, diphenhydramine hydrochloride, promethazine hydrochloride and the like; and vitamins such as alfacalcidol, cobamamide, tocopherol nicotinate, mecobalamin, and the like. One or more of the above-mentioned substances can be used according to purposes of medical treatment.

The medical agent content in the medical-agent-containing layer is not limited to particular amounts and can be adjusted as desired according to the types of medical agents. The weight percent of the medical agent content in the medical-agent-containing layer is commonly 80 wt% or less, preferably 40 wt% or less, and more preferably 30 wt% or less. When the weight percent of the medical agent content exceeds 80 wt%, the strength of the orally administered medication as a film decreases. The lower limit of the medical agent content in the medical-agent-containing layer can be set as desired according to the types of medical agents to be contained in the medical-agent-containing layer and is commonly 0.001 wt% or thereabout.

### (Base substances)

Base substances contained in the medical-agent-containing layer together with medical agents are not limited to particular substances and can be selected as desired according to intended purposes. Examples of the base substances contained in the medical-agent-containing layer include: the cellulose and its derivatives such as crystalline cellulose, carboxymethyl cellulose, hydroxyethyl cellulose, hydroxypropyl cellulose, hydroxypropyl methylcellulose, hydroxypropyl ethylcellulose, methylcellulose, ethylcellulose, cellulose acetate, cellulose acetate phthalate, hydroxypropyl methylcellulose phthalate, hydroxypropyl methylcellulose acetate succinate, carboxymethyl ethylcellulose, and the like; or their pharmaceutically permissible salt such as sodium salts.
Examples of the base substances contained in the medical-agent-containing layer include: starch and its derivatives such as alpha-starch, oxidized starch, sodium carboxymethyl starch, hydroxypropyl starch, dextrin, dextran, and the like; sugar such as sucrose, maltose, lactose, glucose, fructose, pullulan, xanthan gum, cyclodextrin, and the like; sugar alcohol such as xylitol, mannitol, sorbitol, and the like; acrylic acid derivatives such as diethylaminoethyl methacrylate-methacrylic acid copolymer, methacrylic acid-ethyl acrylate copolymer, methacrylate-methyl methacrylate copolymer, ethyl methacrylate-methacrylic acid-trimethylammonium chloride copolymer, diethylaminoethyl methacrylate-chloro methyl methacrylate copolymer, methacrylate- chloro ethyl acrylate copolymer, and the like. Examples of the base substances contained in the medical-agent-containing layer include: shellac; polyvinylacetal diethylaminoacetate; vinyl acetate; polyvinyl alcohol; polyvinylpyrrolidone; natural rubber such as gum arabic, tragacanth gum, and the like, polyglucosaauine such as chitin, chitosan, and the like; proteins such as gelatin, casein, soy protein, and the like; titanium oxide; calcium monohydrogen phosphate; calcium carbonate; talc; stearate; magnesium aluminometasilicate; magnesium silicate; silicic anhydride; and vinyl acetate-vinylpyrrolidone copolymer. One or more of the above-mentioned substances can be used.

Of those base substances, edible polymers are preferred. The edible polymers can be synthesized polymers or natural polymers and are not limited to specific types.

Preferred edible polymers are those soluble in the stomach or in the intestines. Examples of such edible polymers include cellulose and/or its derivatives, especially hydroxypropyl cellulose, hydroxypropyl methylcellulose phthalate, and the like. The hydroxypropyl cellulose and the hydroxypropyl methylcellulose phthalate are especially useful due to their excellent film-forming capabilities.

The edible polymer content in the medical-agent-containing layer is the amount that enables formation of that layer, and the amount can be adjusted as desired according to the types of edible polymers and the like. The weight percent of the edible polymer content in the medical-agent-containing layer is commonly 20 wt% or higher, preferably 60 wt% or higher, and more preferably 70 wt% or higher. A weight percent of the edible polymer content of less than 20 wt% would result in incomplete formation of the medical-agent-containing layer. The upper limit of the edible polymer content in the medical-agent-containing layer is obtained by subtracting the minimum medical agent content in the medical-agent-containing layer from 100 wt% and can be set as desired according to the types of the medical agents and the like.

### (Plasticizers)

Examples of plasticizers include: propylene glycol; polyethylene glycol; glycerin and sorbitol; glycerin triacetate; diethyl phthalate and triethyl citrate; lauric acid; sucrose; and the like, and one or more of those plasticizers can be used.

When a large amount of plasticizers is contained in the medical-agent-containing layer for the purpose of preventing the medical-agent-containing layer 11 from being cracked and chipped, the medical-agent-containing layer 11 may occasionally seep from the edge of the orally administered medication. To prevent such seepages, it is preferred to use as a base substance polyvinylpyrrolidone of a K-value of 70 or higher. The K-value refers to the intrinsic viscosity of a polymer solution and is also called Fikentscher's viscosity constant. Polyvinylpyrrolidone of the K-value of 70 or higher is useful because it serves as an excipient, stabilizer, and binding agent. The use of such polyvinylpyrrolidone of the K-value of 70 or higher prevents the medical-agent-containing layer from being cracked and chipped event if the amount of plasticizers contained in the medical-agent- containing layer is small and also suppresses the water content of the medical-agent-containing layer, thereby improving the stability of the medical agents contained in the medical-agent-containing layer.

As a base substance of the medical-agent-containing layer, polyvinylpyrrolidone of the K-value of 70 or higher can be used alone. It is also possible to use one of the above-mentioned base substances in addition to that polyvinylpyrrolidone. In this case, the one of the base substances can be polyvinylpyrrolidone of the K-value of less than 70. It is preferred that hydroxypropyl cellulose or hydroxypropyl methylcellulose phthalate, both of which are excellent in film-forming capabilities, is used in combination.

The entire base substance content in the medical-agent-containing layer 11 is the amount that enables formation of that layer 11, and the amount can be adjusted as desired according to the types of base substances and the like. The weight percent of the base substance content in the medical-agent-containing layer 11 is commonly 30 wt% or higher, preferably 60 wt% or higher, and more preferably 70 wt% or higher. A weight percent of the entire base substance content of less than 30 wt% would result in incomplete formation of the medical-agent-containing layer 11. The upper limit of the entire base substance content in the medical-agent-containing layer 11 is obtained by subtracting the minimum medical agent content in the medical-agent-containing layer 11 from 100 wt% (or obtained, when the medical-agent-containing layer 11 also contains plasticizers, by subtracting the minimum content of the medical agents and plasticizers contained in the medical-agent-containing layer 11 from 100 wt%) and can be set as desired according to the types of medical agents, the types of plasticizers, and the like. For instance, when the minimum medical agent content in the medical-agent-containing layer 11 is 0.01 wt%, the upper limit of the entire base substance content in the medical-agent-containing layer 11 is 99.99 wt%. When the minimum medical agent content and the minimum plasticizer content in the medical-agent-containing layer 11 are 0.01 wt% and 2 wt%, respectively, the upper limit of the entire base substance content in the medical-agent-containing layer 11 is 97.99 wt%.

In the case, the amount of the plasticizers contained in the medical-agent-containing layer 11 can be adjusted as desired according to the amount of polyvinylpyrrolidone of the K-value of 70 or higher contained in the medical-agent-containing layer 11 and the like. The amount of the plasticizers in the medical-agent-containing layer 11 is commonly from 2 to 25 wt%, preferably from 4 to 21 wt%, and more preferably from 6 to 17 wt%. When the plasticizer content exceeds 25 wt%, the medical-agent-containing layer 11. may seep from the edge of the orally administered medication 1. When, on the other hand, the plasticizer content is less than 2 wt%, the plasticizers cannot function well as such.

When the medical-agent-containing layer 11 contains plasticizers, it is preferred that the content of polyvinylpyrrolidone of the K-value of 70 or higher in the medical-agent-containing layer 11 be 30 wt% or higher and that the amount of plasticizers in the medical-agent-containing layer 11 be from 2 to 25 wt%. This effectively prevents the medical-agent-containing layer 11 from being cracked and chipped and from seeping from the edge of the orally administered medication 1.

When the medical-agent-containing layer 11 contains plasticizers, the amount of the medical agents contained in the medical-agent -containing layer 11 can be adjusted as desired according to the types of the medical agents. The medical agent content in the medical-agent-containing layer 11 is 70 wt% or less, preferably 40 wt% or less, and more preferably 30 wt% or less. When the medical agent content exceeds 70 wt%, the film strength decreases. The lower limit of the medical agent content can be set as desired according to the types of medical agents to be contained in the medical-agent-containing layer 11 and is commonly 0.01 wt% or thereabout.

### (Water-swellable gel forming agents)

Water-swellable gel forming agents are not limited to particular substances as long as they are capable of swelling in response to water and forming a gel. Examples of water-swellable gel forming agents include: carboxyvinyl polymers; starch and its derivatives; agar; alginic acid; arabinogalactan; galactomannan; cellulose and its derivatives such as carboxymethyl cellulose and the like; carrageenans; dextran; tragacanth; gelatin; pectin; hyaluronic acid; gellan gum; collagen; casein; xanthan gum; glucomannan; and the like. Such gel forming agents may or may not be cross-linked. Preferred gel forming agents are cross-linked carboxyvinyl polymers, and cross-linked polyacrylic acid is particularly preferred. The use of the cross-linked carboxyvinyl polymers or more preferably the cross-linked polyacrylic acid imparts suitable gel strength to the gel forming layers 12 and 13 upon swelling without affecting the turn-forming capabilities of film-forming agents. Because polyvinylpyrrolidone of the K-value of 70 or higher interacts with the cross-linked polyacrylic acid, imparting suitable gel strength, the use of such polyvinylpyrrolidone as a base substance is also preferred.

Cross-linking is achieved by cross-linking agents, which are selected according to the types of molecules to be cross-linked. Of the above-mentioned water-swellable gel forming agents, carboxyvinyl polymers, alginic acid, pectin, carboxylcellulose, glucomannan, and the like can be cross-linked, for example, by polyvalent metal compounds. Examples of polyvalent metal compounds include: calcium chloride, magnesium chloride, aluminum chloride, aluminum sulfate, aluminum potassium sulfate, iron chloride alum, ammonium alum, ferric sulfate (II), aluminum hydroxide, aluminum silicate, aluminum phosphate, iron citrate, magnesium oxide, calcium oxide, zinc oxide, zinc sulfate, and the like. When polyvinylpyrrolidone of the K-vale of 70 or higher is to be used as a base substance, the use of trivalent metal compounds of such polyvalent metal compounds increases the cross-linkage degree of polyacrylic acid and enables formation of a gel with high gel-strength during interaction between the cross-linked polyacrylic acid and the polyvinylpyrrolidone of the K-value of 70 or higher.

### (Filna-forming agents)

Film-forming agents are not limited to particular types as long as they have film-forming capabilities. Examples of film-forming agents include: polyvinyl alcohol; polyvinylpyrrolidone; polyvinyl acetate; polyvinyl acetate phthalate; hydroxyalkyl cellulose such as hydroxypropyl cellulose, hydroxypropyl methylcellulose, hydroxymethyl cellulose, and hydroxyethyl cellulose; alkylcellulose such as methylcellulose and ethylcellulose; carboxyalkyl cellulose such as carboxymethyl cellulose (meth)acryl acid and its esters; xanthan gum; carrageenans; alginic acid; and the like.

Film-forming agents are preferably water-soluble. If so, water can permeate the water-swellable gel forming layers with ease, facilitating swelling of the gel forming layers and gel formation inside the oral cavity.

Examples of water-soluble film-forming agents include: polyvinyl alcohol; hydroxyalkyl cellulose such as hydroxypropyl cellulose, hydroxypropyl methylcellulose, methylcellulose, and the like; polyvinylpyrrolidone; xanthan gum; carrageenans; alginic acid; and the like. Of these, polyvinyl alcohol, which functions also as a masking agent, or the like is preferred.

The film-forming agent content in the gel forming layers 12 and 13 can be adjusted as desired according to the types of film-forming agents and is preferably from 30 to 85 wt%.

### (Masking agents)

Examples of masking agents include: agents that acidulate such as citric acid, tartaric acid, fumaric acid, and the like; sweeting agents such as saccharin, aspartame, stevioside, glycyrrhizic acid, sucrose, fructose, mannitol, and the like; refreshing agents such as menthol, peppermint oil, peppermint, spearmint, and the like; natural or synthesized fragrant substances; and the like. One or more of those substances can be selected for use.

### (Colorants)

Examples of colorants include edible lake colorants and the like.

### (Preservatives)

Examples of preservatives include: methyl hydroxybenzoate; propyl hydroxybenzoate; and the like.

As stated above, it is preferred to use polyvalent metal compounds as cross-linking agents since the high gel strength that results when the gel forming layers swell in response to water makes the orally administered medication easy to swallow. However, when the gel forming layers are formed with the use of water-swellable gel forming agents cross-linked by polyvalent metal ions, the viscosity of the coating liquid to form the gel forming layers may increase, making it difficult to coat the coating liquid. Thus, by having not the gel forming layers but the medical-agent-containing layer include polyvalent metal compounds that generate polyvalent metal ions and by having the gel forming layers include gel forming agents that are to be cross-linked by the polyvalent metal ions, it becomes possible to form an orally administered medication whose gel forming layers have sufficient strength. As the polyvalent metal compounds in this case, the above-mentioned polyvalent metal compounds can be used.

When polyvalent metal compounds are included in the medical-agent-containing layer, the above-mentioned base substances can be used as the base substances of that layer. It is preferred to use base substances that are without ion functional groups which could react with polyvalent metal ions generated from the polyvalent metal compounds. The reason is that if the base substances of the medical-agent-containing layer include such ion functional groups which could react with polyvalent metal ions, the polyvalent metal ions generated from the polyvalent metal compounds are utilized for the cross-linkage of the base substances of the medical-agent-containing layer when the water-swellable gel forming layers swell in response to water such as saliva and the like in the oral cavity of the patient. This may result in insufficient cross-linkage of the water-swellable gel forming agents contained in the gel forming layers.

Examples of the ion functional groups which could react with polyvalent metal ions include: carboxyl groups; sulfonate groups; phosphate groups; phenolic hydroxyl groups; and the like. Among the above-mentioned base substances, the ones without the ion functional groups which could react with polyvalent metal ions are: for example, polyvinyl alcohol; polyvinylpyrrolidone; cellulose and its alkyl esters; starch and its derivatives; polyvinyl acetate; polyethylene glycol; sugar; (meth)acrylate-alkyl esters copolymer; vinyl acetate-vinylpyrrolidone copolymer; and the like.

When polyvalent metal compounds are included in the medical-agent-containing layer, the total amount of the base substances contained in the medical-agent-containing layer 11 is the amount that enables formation of that layer 11., and the amount can be adjusted as desired according to the types of base substances and the like. The mass percent of the base substance content in the medical-agent-containing layer 11 is commonly 30 mass% or higher, preferably 60 mass% or higher, and more preferably 65 mass% or higher. A mass percent of the base substance content of less than 30 mass% would result in incomplete formation of the medical-agent" containing layer 11. The upper limit of the base substance content in the medical-agent-containing layer can be set as desired according to the amount of medical agents.

When polyvalent metal compounds are included in the medical-agent-containing layer, the medical agent content in the medical-agent-containing layer 11 is not limited to particular amounts and can be adjusted as desired according to the types of medical agents. The mass percent of the medical agent content in the medical-agent-containing layer 11 is commonly 70 mass% or less, preferably 40 mass% or less, and more preferably 35 mass% or less. When the mass percent of the medical agent content exceeds 70 mass%, the film strength of the orally administered medication 1 decreases. The lower limit of the medical agent content in the medical-agent-containing layer can be set as desired according to the types of medical agents to be contained in the medical-agent-containing layer 11 and is commonly 0.01 mass% or thereabout.

With the above contents, the water-swellable gel forming agents contained in the medical-agent-containing layer can be cross-linked by polyvalent metal ions during swelling of the gel forming layers in response to water. This eliminates the need to include sufficiently-cross-linked water-swellable gel forming agents in the gel forming layers in advance. Accordingly, in forming the gel forming layers, the viscosity of their coating liquid does not increase, causing no difficulty in coating, and a gel with sufficient strength can be obtained.

Water-swellable gel forming agents which can be contained in the water-swellable gel forming layers 12 and 13 and cross-linked by polyvalent metal ions are not particularly limited. Examples of such gel forming agents are: polyacrylic acid, polymethacrylic acid, carboxyvinyl polymers, alginic acid, pectin, carboxymethyl cellulose, glucomannan, and the like. One or more of those substances can be selected for use.

It is also possible for the water-swellable gel forming layers 12 and 13 to include water-swellable gel forming agents that cannot be cross-linked by polyvalent metal ions. Examples of such gel forming agents that cannot be cross-linked by polyvalent metal ions include: starch and its derivatives; agar; carrageenans; gellan gum; gelatin; collagen; hydroxypropyl cellulose; xanthan gum; and the like. One or more of those substances can be selected for use.

The amount of the water-swellable gel forming agents which are contained in the water-swellable gel forming layers 12 and 13 and can be cross-linked by polyvalent metal ions can be adjusted according to the types of the water-swellable gel forming agents and the like. The content of the water-swellable gel forming agents in the gel forming layers 12 is commonly from 5 to 90 mass%, preferably from 10 to 80 mass%, and more preferably from 15 to 70 mass%.

The amount of polyvalent metal compounds contained in the medical-agent-containing layer 11 can be adjusted as desired according to the amount of water-swellable gel forming agents which are contained in the gel forming layers 12 and 13 and can be cross-linked by polyvalent metal ions. The parts by mass of such polyvalent metal compounds in the medical-agent-containing layer 11 are commonly 0.01 to 10 parts by mass with respect to 1 part by mass of the water-swellable gel forming agents which can be cross-linked by polyvalent metal ions, preferably 0.03 to 8 parts by mass, and more preferably 0.05 to 5 parts by mass. When the polyvalent metal compounds are less than 0.01 parts by mass, the amount of the polyvalent metal ions generated from the polyvalent metal compounds is not sufficient when the gel forming layers swell in response to water such as saliva and the like inside the oral cavity of the patient. This results in insufficient cross-linkage of the water-swellable gel forming agents contained in the gel forming layers. When, on the other hand, the polyvalent metal compounds are more than 10 parts by mass, the following may result: reduction in the medical agent content in the medical-agent-containing layer 11, perception by the patient of the taste of the polyvalent metal compounds (bitterness or the like), plasticization of the medical-agent-containing layer 11 due to the moisture-absorbing action by the polyvalent metal compounds, reduction in the stability of the medical-agent-containing layer 11, and the like.

Intermediate layers may be provided between the gel forming layers and the medical-agent-containing layer, and polyvalent metal compounds may be included in the intermediate layers. In this case, the intermediate layers can have the same base substances as those of the medical-agent-containing layer in order to maintain the polyvalent metal compounds. By providing the water-swellable gel forming layers 12 and 13 as the outermost layers of the orally administered medication 1 with each stacked directly on the intermediate layers, the polyvalent metal compounds contained in the intermediate layers can be ionized to generate polyvalent metal ions when the gel forming layers 12 and 13 swell in response to water such as saliva and the like to form a gel inside the oral cavity of the patient. The water-swellable gel forming agents contained in the gel forming layers 12 and 13 are cross-linked by those polyvalent metal ions. Accordingly, a gel with sufficient strength can be formed without the use of sufficiently-cross-linked water-swellable gel forming agents in the gel forming layers 12 and 13 in advance.

Further, the surfaces of the water-swellable gel forming layers 12 and 13 may be coated with polyvalent metal compounds. The polyvalent metal compounds are such as those included in the medical-agent-containing layer 11 of the orally administered medication 1. Note that the medical-agent-containing layer 11 of the orally administered medication 1 may include polyvalent metal compounds, but does not necessarily have to.

When the gel forming layers 12 and 13 of the orally administered medication 1 swell in response to water such as saliva and the like to form a gel inside the oral cavity of the patient, the polyvalent metal compound coatings on the water-swellable gel forming layers 12 and 13 are ionized to generate polyvalent metal ions. The water-swellable gel forming agents contained in the water-swellable gel forming layers 12 and 13 are cross-linked by those polyvalent metal ions. Accordingly, a gel with sufficient strength can be formed without the use of sufficiently-cross-linked water-swellable gel forming agents in the water-swellable gel forming layers 12 and 13 in advance.

The thickness of each of the polyvalent metal compound coatings on the water-swellable gel forming layers 12 and 13 can be adjusted as desired within the thickness range in which the orally administered medication can be administered orally. The thickness is preferably from 1 to 500 µm and more preferably from 5 to 100 µm. When the thickness of each of the polyvalent metal compound coatings is less than 1 µm, uniform thickness is difficult to achieve, and the amount of the polyvalent metal compounds in each coating is not large enough to obtain sufficient gel strength. When, on the other hand, the thickness of each of the polyvalent metal compound coatings exceeds 500 µm, sufficient gel formation cannot be achieved with only a small amount of water such as saliva and the like upon intake, causing intake difficulties.

when the gel forming layers swell in response to water such as saliva and the like to form a gel inside the oral cavity of the patient, the medical agents contained in the medical-agent-containing layer may occasionally dissolve and seep out of the orally administered medication due to the water reaching the medical-agent-containing layer. In such cases, the taste, smell, and the like of the medical agents cannot be masked sufficiently even if some of the above-mentioned masking agents are contained. For the purpose of protecting the medical agents in the medical-agent-containing layer from water and of preventing the medical agents from seeping out of the orally administered medication, it is thus preferred that poorly water-soluble polymers be contained in the medical-agent-containing layer as its base substances. The use of the poorly water-soluble polymers leads to more effective masking of the taste, smell, and the like of the medical agents.

In this case, it is preferred that intermediate layers including polyvinylpyrrolidone be provided between the gel forming layers and the anedical-agent-containing layer in order to increase the gel strength when the gel forming layers swell in response to water. The reason is that the water-swellable gel forming agents contained in the gel forming layers interact with the pyrrolidone contained in the intermediate layers, enabling formation of a gel with higher strength.

Poorly water-soluble polymers which can be contained in the medical-agent-containing layer 11 are not particularly limited. Examples of the poorly water-soluble polymers include: polyvinyl alcohol; ethylcellulose; hydroxypropyl methylcellulose; acetate succinate; hydroxypropyl methylcellulose phthalate; acetylcellulose; cellulose acetate phthalate; carboxymethyl cellulose; hydroxypropyl starch; dimethylaminoethyl (meth)acrylate- (meth)acrylate copolymer; (meth)acrylate-ethyl acrylate copolymer; (meth)acrylate-methyl (meth)acrylate copolymer; ethyl (meth)acrylate-trimethylammonium chloride (meth)acrylate copolymer; dimethylaminoethyl (meth)acrylate-methyl chloride (meth)acrylate copolymer; (meth)acrylate- ethyl chloride acrylate copolymer; polyvinylacetal diethylaminoacetate; polyvinyl acetate; shellac; and the like. One or more of those substances can be selected for use. It is preferred to use polyvinyl alcohol alone or use other poorly water-soluble polymers in addition to the polyvinyl alcohol. Since the polyvinyl alcohol, as stated above, effectively masks the taste, smell, and the like of medical agents, its use in the medical-agent-containing layer 11 effectively prevents the medical agents in the medical-agent-containing layer 11 from seeping out of the orally administered medication 1 and also enables effective masking of the taste, smell, and the like of medical agents contained in the medical-agent.containing layer 11.

The amount of the poorly water-soluble polymers contained in the medical-agent-containing layer 11 is commonly 10% or higher by mass of the medical-agent-containing layer 11, preferably 15% or higher by mass, and more preferably 20 % or higher by mass. When the amount of the poorly water-soluble polymers contained in the medical-agent-containing layer 11 is in those ranges, medical agent seepages from the orally administered medication 1 can be effectively prevented. The upper limit of the amount of the poorly water-soluble polymers contained in the medical-agent-containing layer 11 is the amount obtained by subtracting the minimum medical agent content from 100 mass%. For instance, when the minimum medical agent content in the medical-agent-containing layer 11 is 0.01 mass%, the upper limit of the content of the poorly water-soluble polymers is 99.99 mass%.

The medical-agent-containing layer 11 may include as its base substances poorly water-soluble polymers only, but it may also include water-soluble polymers in addition to the poorly water-soluble polymers. Examples of the water-soluble polymers include: polyvinylpyrrolidone; vinyl acetate-vinylpyrrolidone copolymer; polyethylene glycol; hydroxypropyl cellulose; hydroxypropyl methylcellulose; pullulan, xanthan gum; gum arabic; starch; gelatin; dextrin; dextran; and the like. The amount of the water-soluble polymers contained in the medical-agent-containing layer 11 is commonly 50% or less by mass of the medical-agent-containing layer 11, preferably, 30% or less by mass, and more preferably 20% or less by mass. The lower limit of the content of the water-soluble polymers is zero.

When the medical-agent-containing layer 11 includes poorly water-soluble polymers, preferred intermediate layers are those including polyvinylpyrrolidone, as stated above. The intermediate layers may include only polyvinylpyrrolidone or may also include base substances that maintain the polyvinylpyrrolidone in the intermediate layers in a desired state. The amount of the polyvinylpyrrolidone in the intermediate layers is commonly 10% or higher by mass of the intermediate layers, preferably 20% or higher by mass, and more preferably 40% or higher by mass. The K-value of the polyvinylpyrrolidone contained in the intermediate layers is preferably 90 or higher although a K-value of more than 30 suffices to form a gel with enough strength.

The thickness of an intermediate layer is from 1 to 80 µm and preferably from 10 to 50 µm. If the thickness is in those ranges, the orally administered medication 1 can be administered orally.

Each of the above-mentioned orally administered medication according to the invention may include adhesive layers which are different from the above intermediate layers and arranged between the layers. The adhesives contained in the adhesive layers are not particularly limited as long as they are pharmaceutically permissible ones. Adhesives that exert their adhesive properties when used with solvent contained in the adhesives are for example, carboxyvinyl polymers; polyacrylic acid and its pharmaceutically permissible nontoxic salts such as sodium polyacrylate and the like; acrylic acid copolymers and their pharmaceutically permissible salts; hydrophilic cellulose derivatives such as carboxymethyl cellulose, sodium salt, and the like; pullulan; povidone; gum karaya; pectin; xanthan gum; tragacanth; alginic acid; gum arabic; acid polysaccharides, their derivatives, or their pharmaceutically permissible salts; and the like. One or more of those substances can be selected for use. Further, adhesives that exert their adhesive properties when heated (thermal adhesives) include: for example, vinyl acetate; homopolymers such as polyvinylpyrrolidone and the like; vinyl acetate-vinylpyrrolidone copolymer; and the like. One or more of those substances can also be selected for use.

The thickness of the adhesive layer can be adjusted as desired within the thickness range in which the orally administered medication can be administered orally. The thickness is preferably from 1 to 50 µm and more preferably from 10 to 30 µm. A thickness of the adhesive layer of less than 1 µm may result in adhesion failure. On the other hand, a thickness of more than 50 µm may hinder the swelling of the gel forming layers that occurs in response to saliva and the like during intake of the orally administered medication and at the same time may cause intake discomfort when the adhesives contained in the adhesive layer are not soluble in water.

A manufacturing method for the orally administered medication 1 is discussed below.

First, coating liquids for the gel forming layers 12 and 13 and the medical-agent-containing layer 11 are prepared. Next, the coating liquid for the gel forming layers is applied onto a base film, and then dried. The base film is provided with concave portions so that desired convex portions can be formed on the surface of the gel forming layer 12 of the orally administered medication 1. Thus, by applying the coating liquid onto the base film, the gel forming layer having desired convex portions formed on its surface can be obtained. For example, the use of a base film having on its surface an embossed pattern that has the reversed concave-convex relationship between the concave and convex portions of FIGS. 2A and 2B enables easy formation of convex portions on the order of micrometers. After another gel forming layer is formed, the coating liquid for the medical-agent-containing layer 11 is applied onto the surface of each gel forming layer (the surface is opposite the surface on which the convex portions of the gel forming layer are formed) and then dried to form medical-agent-containing layers 11. The resultant two laminate bodies each of which has the laminated medical-agent-containing layer/ gel forming layer/base film in the described order, are glued and pressed together with the medical-agent-containing layers 11 facing each other. Thereafter, the base films are peeled and removed, and the rest is cut into a particular shape, thereby forming the orally administered medication 1. The thus-formed orally administered medication 1 has convex portions on both of its surfaces. It should be noted that the base films may be left until dosage without removing.
The above method applies when convex portions are formed on both the surfaces of the orally administered medication 1. When convex portions need only be formed on either of the surfaces of the orally administered medication 1, a flat base film can be used as the base film for the side on which convex portions are not formed.

The base films with the concave portions can be manufactured by applying embossing or the like to form concave portions on plastic base films comprising: polyester such as polyethylene terephthalate, polyethylene naphthalate, and the like; polyolefin such as polypropylene, polymethylpentene, and the like; or thermoplastic resin such as polycarbonate and the like. If necessary, at least either of the surfaces of a base film can be subjected to peeling.

In the above manufacturing method, convex portions are formed on the orally administered medication 1 with the use of the base films with concave portions. Alternatively, compression molding or partial coating can be employed to form convex portions in a desired pattern.

When the surfaces of the water-sweeable gel forming layers 12 and 13 are to be coated with polyvalent metal compounds, a solution containing polyvalent metal compounds that can generate polyvalent metal ions (the solvent is, for example, purified water, ethanol, or the like) is applied or sprayed onto base films that have concave and convex portions for the purpose of forming desired convex portions, and then dried. Thereafter, the coating liquid for the water-swellable gel forming layers is applied or sprayed onto the polyvalent metal compound coatings, and the films are then dried, thereby forming the water-swellable gel forming layers 12 and 13. Alternatively, the surfaces of the water-swellable gel forming layers 12 and 13 can be coated with polyvalent metal compounds by applying or spraying a solution containing polyvalent metal compounds that can generate polyvalent metal ions onto the surfaces of the water-swellable gel forming layers 12 and 13, and then drying. The above-mentioned solutions can contain water-soluble polymers, examples of which include: the film-forming agents listed above, the base substances of the medical-agent-containing layer 11 listed above, and the like.

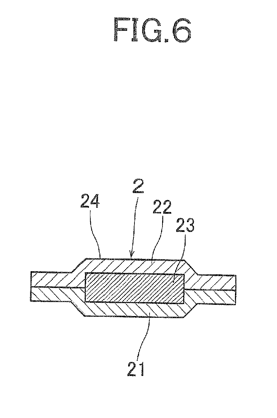

With reference now to FIG. 6, an orally administered medication according to the second embodiment of the invention is described below. The orally administered medication 2 of FIG. 6 includes a first gel forming layer 21, a second gel forming layer 22, and a medical-agent-containing layer 23 which is smaller than the first and second gel forming layers 22 and 22. The orally administered medication 2 is formed by sandwiching the medical-agent-containing layer between the gel forming layers and by joining the gel forming layers at their peripheral edges so that the medical-agont-containing layer 23 is covered with the first and second gel forming layers 21 and 22. In this case, too, a surface 24 of each gel forming layer is provided with convex portions so that the contact area of the orally administered medication 2 with the oral mucous membrane of the patient can be reduced. Preferably, the convex portions are formed in such patterns as shown in FIGS. 2A to 5.

The orally administered medication 2 is preferably formed from the same substances as those of the aforementioned orally administered medication 1 and can include intermediate layers and adhesive layers.

According to a method for manufacturing the second orally administered medication 2 of the invention, coating liquids for the first and second gel forming layers 21 and 22 and the medical-agent-containing layer 23 are prepared first. Next, the coating liquid for the first gel forming layer 21 is applied onto a first base film having concave portions so that desired convex portions can be formed on the surface 24 of the first gel forming layer 21, and then dried to form the first gel forming layer having convex portions on its surface. Thereafter, the coating liquid for the medical-agent-containing layer 23 is printed onto the first gel forming layer 21 by a known method such as screen printing or the like such that the medical-agent-containing layer 23 is formed smaller than the first gel forming layer 21, and then dried to form the medical-agent-containing layer 23. Meanwhile, the coating liquid for the second gel forming layer 22 is applied onto a second base film having concave portions so that desired convex portions can be formed on the surface 24 of the second gel forming layer 22, and then dried to form the second gel forming layer having convex portions on its surface. Next, the peripheral edges of the first and second gel forming layers 21 and 22 are joined together, thus forming the orally administered medication 2. Methods for joining the peripheral edges include: for example, (i) a method in which the peripheral edge of the first gel forming layer 21 is wetted with water to cause the peripheral edge to gelate, the second gel forming layer 22 is pressed onto the gelated peripheral edge, and then dried to joint; (ii) a method in which the peripheral edges of the first and second gel forming layers 21 and 22 are directly joined together by thermal fusion bonding; and (iii) a method in which an adhesive layer(s) is/are formed on either the first gel forming layer 21 or the second gel forming layer 22 or both, and the gel forming layers 21 and 22 are jointed by the adhesive layer(s).
The above method applies when convex portions are formed on both the surfaces of the orally administered medication 2. When convex portions need only be formed on either of the surfaces of the orally administered medication, a flat base film can be used as the first base film or the second base film.

An orally administered medication according to the third embodiment of the invention is one with convex portions that is formed by shaping the film-like orally administered medication 1 into accordion folds. In the case, the medication has a much smaller contact area because only the accordion folds of the medication come into contact with the oral mucous membrane. Thus, even if attached to the oral mucous membrane, the medication can be detached with ease.

### [Example 1]

The invention is discussed in further detail below with illustration of various examples. Note that the scope of the invention is not limited to those examples.

### (1) Preparation of a coating liquid for a gel forming layer:

A calcium chloride of 0.3 g (trade name "calcium chloride H," Tomita Pharmaceutical Co., Ltd.) was added as a cross-linking- agent to a purified water of 300 g, and the water was stirred for dissolution of the calcium chloride. Next, a polyacrylic acid of 11.3 g (trade name "Carbopol 974P," Noveon, Inc.), which serves as a water-swellable gel forming agent, was slowly added to the resultant solution with the solution stirred and was dissolved by stirring. Then, a polyvinyl alcohol of 33.8 g (trade name "Gohsenol EG-05T," Nippon Synthetic Chemical Industry Co., Ltd), which serves as a film-forming agent, was slowly added to the solution with the solution stirred and was completely dissolved by stirring the solution for an hour and a half while heating the solution at 70 degrees Celsius in a water bath. Thereafter, the solution was cooled to room temperature, a concentrated glycerin of 4 g (trade name "concentrated glycerin according to the Japanese Pharmacopoeia," Asahi Denka Co., Ltd.) was added as a plasticizer to the solution, and the solution was stirred for 5 minutes, thereby completing the coating liquid for the gel forming layer.

### (2) Preparation of a coating liquid for a medical-agent-containing layer:

A famotidine of 2.5 g (gastric ulcer medication), which serves as a medical agent, and a titanium oxide of 0.6 g (trade name "Tipaque Cur-50," Ishihara Sangyo Kaisha, Ltd.), which serves as a base substance, were added to a purified water of 53.7 g and sufficiently dispersed therein with the use of a homogenizer. Next, a polyvinylpyrrolidone of 13.8 g (trade name "Plasdone K-90," ISP Japan Ltd.), which also serves as a base substance, was added to the solution with the solution stirred and was completely dissolved by stirring the solution for about half an hour. Then, the solution was cooled to room temperature, a concentrated glycerin of 4 g (trade name "concentrated glycerin according to the Japanese Pharmacopoeia," Asahi Denka Co., Ltd.) was added as a plasticizer to the solution, and the solution was stirred for 5 minutes, thereby completing coating liquid for the medical-agent-containing layer.

### (3) Formation of a gel forming layer:

After coating liquid for the gel forming layer was sufficiently defoamed, it was applied evenly with the use of an applicator on a polyethylene-terephthalate (PET) base film embossed by a given method (the film had concave portions so that the convex portions of FIGS. 2A and 2B could be formed). The coating liquid for gel forming layer was then dried at 80 degrees Celsius for 5 minutes, thus forming the water-swellable gel forming layer. The thickness of the obtained gel forming layer including its convex portions was 38 µm.

### (4) Formation of a medical-agent-containing layer:

After coating liquid for the medical-agent-containing layer was sufficiently defoamed, it was applied evenly on the gel forming layer with the use of an applicator whose gap was adjusted such that the coating amount after drying would be 50 g/m² (50 µm thick). The coating liquid for the medical-agent-containing layer was then dried at 80 degrees Celsius for 5 minutes, thus forming the medical-agent-containing layer. Thus, two of a sheet having the laminated base film, the gel forming layer, and the medical-agent-containing layer in the described order was obtained.

The two sheets obtained as described above comprising the laminated base film, the gel forming layer, and the medical-agent-containing layer in the described order were then thermally joined together with their medical-agent-containing layers facing each other by pressing them at 1 kgf/cm² for 1 second in a 100-degrees-Celsius environment. After the embossed PET base films were detached from the gel forming layers, a 15-mmφ die was used to shape an orally administered medication having the laminated gel forming layer/medical-agent containing layer/ gel forming layer and having convex portions on the surfaces of the outermost gel forming layers, which convex portions were formed by the embossed patterns of the base films being transferred.

### [Example 2]

An orally administered medication was manufactured in the same manner as Example 1 except that base films were used that had such an embossed pattern as would allow the convex portions shown in FIGS. 3a and 3B to be formed on the entire surface of the gel forming layers. The thickness of each of the gel forming layers including its convex portions was 80 µm.

### [Example 3]

An orally administered medication was manufactured in the same manner as Example 1 except that base films were used that had such an embossed pattern as would allow the convex portions of FIG. 5 to be formed on the entire surface of the gel forming layers. The thickness of each of the gel forming layers including its convex portions was 205 µm.

### [Comparative Example 1]

An orally administered medication was manufactured in the same manner as Example 1 except that flat PET base films with no embossed pattern were used.

### (Evaluation of the adherability of the orally administered medications with respect to the oral mucous membrane)

The orally administered medications manufactured in Examples 1 to 3 and Comparative Example 1 were administered to five human subjects without water so that the medications could easily touch the upper jaws of the subjects, and the adherability of the orally administered medications with respect to the upper jaws was evaluated based on the following five-point scale. The result is shown in Table 1.
1: The entire one surface of the orally administered medication was attached to the upper jaw and could not be detached with ease.
2: One surface of the orally administered medication was partially attached to the upper jaw, and the attached portion could not be detached with ease.
3: Although one surface of the orally administered medication was partially attached to the upper jaw, the subject could detach the attached portion easily with subject's tongue and could take the medication.
4 Although one surface of the orally administered medication was partially attached to the upper jaw, the attached portion fell off quickly and the subject could take the medication.
5: The subject could take the orally administered medication quickly without the medication attached to the upper jaw.

**[Table 1]**

| | Embossed pattern | Subject 1 | Subject 2 | Subject 3 | Subject 4 | Subject 5 | Average |
|---|---|---|---|---|---|---|---|
| Example 1 | FIG. 2 | 4 | 3 | 3 | 4 | 3 | 3.4 |
| Example 2 | FIG. 3 | 4 | 5 | 5 | 4 | 5 | 4.6 |
| Example 3 | FIG. 5 | 5 | 4 | 5 | 5 | 5 | 4.8 |
| Comparative Example 1 | Flat | 1 | 1 | 2 | 2 | 2 | 1.6 |

Table 1 reveals that the orally administered medications of Examples 1 to 3 with convex portions were high in low adherability with respect to the oral mucous membrane and easy to swallow even without water. In contrast, the orally administered medication of Comparative Example 1 was found to be difficult to take because of its tendency to be attached to the oral mucous membrane.

## Claims

1. A film-like orally administered medication comprising at least a medical-agent-containing layer, wherein a convex portion is formed on at least one surface of the orally administered medication.

2. The orally administered medication of claim 1, comprising a gel forming layer as an outermost layer of the orally administered medication, wherein the convex portion is formed on the surface of the gel forming layer.

3. The orally administered medication of claim 1 or 2, wherein the convex portion has at least one type of shape selected from among tapers, columns, hemispheres, and frustums.

4. The orally administered medication of any one of claims 1 to 3, wherein the height of the convex portion is in the range from 10 to 5,000 µm.

5. The orally administered medication of any one of claims 1 to 4, wherein the convex portion is provided in the form of a multiplicity of convex portions on at least one surface of the orally administered medication.

6. The orally administered medication of any one of claims 1 to 5, wherein the medical-agent-containing layer includes a medical agent and a base substance.

7. The orally administered medication of any one of claims 1 to 6, wherein the thickness of the medical-agent-containing layer is in the range from 0.1 to 1,000 µm.

8. The orally administered medication of any one of claims 2 to 7, wherein the gel forming layer includes a water-swellable gel forming agent and a film-forming agent.

9. The orally administered medication of any one of claims 2 to 8, wherein the thickness of the gel forming layer including the thickness of the convex portion is in the range from 20 to 6,000 µm.

10. A method for manufacturing an orally administered medication, comprising the steps of:
applying a coating liquid for a gel forming layer onto a base film having a concave portion formed thereon and drying;
applying a coating liquid for a medical-agent-containing layer onto the gel forming layer and drying, thus forming a laminated body having the laminated medical-agent-containing layer/ gel forming layer/ base film in the described order;
preparing two of the laminated body and joining and pressure-bonding the two laminated bodies together with the medical-agent-containing layers thereof facing each other; and
removing the base films and cutting the rest into a predetermined shape to form the orally administered medication.

11. A method for manufacturing an orally administered medication, comprising the steps of:
applying a coating liquid for a first gel forming layer onto a first base film having a concave portion formed thereon and drying to form the first gel forming layer;
printing a coating liquid for a medical-agent-containing layer onto the first gel forming layer and drying to form the medical-agent-containing layer that is smaller than the first gel forming layer;
applying a coating liquid for a second gel forming layer onto a second base film having a concave portion formed thereon and drying to form the second gel forming layer; and
bonding peripheral edges of the first and second gel forming layers together to form the orally administered medication.
